# EUROPEAN PATENT SPECIFICATION

(11) **EP 1 455 870 B1**
(45) Date of publication and mention of the grant of the patent: **08.08.2007**
(21) Application number: 02781170.2
(22) Date of filing: 03.12.2002
(51) Int. Cl.: A61M 5/315

(54) **A MEDICAL DELIVERY SYSTEM**
MEDIZINISCHE ABGABEVORRICHTUNG
SYSTEME D'APPORT MEDICAL

(30) Priority: 06.12.2001 DK 200101814
(43) Date of publication of application: 15.09.2004
(73) Proprietor: NOVO NORDISK A/S, 2880 Bagsvaerd (DK)
(72) Inventor: NIELSEN, Lars, Korsbjerg, DK-3460 Birkerød (DK); KRISTENSEN, Lars, Thougaard, DK-3670 Veksø (DK); STENTOFT, Erik, DK-1810 Frederiksberg C (DK); MELANDER, Matias, DK-1705 Copenhagen V (DK)
(86) International application number: PCT/DK2002/000813
(87) International publication number: WO 2003/047668

(56) References cited:
- WO-A-98/11927
- US-A- 5 222 942
- US-A- 5 928 202
- US-B1- 6 206 859

## Description

### The Technical field of the invention:

The invention relates to a medication delivery device for delivering medicine or other fluent material into a mammal body, and especially to a medication delivery device where a plunger located in a tubular cartridge is connected to a plunger rod.

### Description of related art:

Medication delivery devices are widely used throughout the health sector for delivering fluent medicine into mammal bodies through a conduit inserted in the body. In general two types of medication delivery devices exist. The first type being an injection device, which injects a predetermined dose of the fluent medicine into a mammal body whenever the injection device is activated. The other type being an infusion device, which delivers a constant rate of medicine into a mammal body either by gravity or by pressure, which pressure are usually generated by an electrical motor.

The fluent medicine is usually contained in a cartridge, which can be either a fillable cartridge or a pre-filled disposable cartridge. Such known cartridges are e.g. provided as a cylindrical barrel having a penetrable membrane at a distal end and a movable plunger at the opposite proximal end. A conduit penetrating the membrane is mounted at the distal end.

When the plunger is moved towards the membrane the fluent medicament contained in the cartridge is pressed out through the conduit. When the conduit is mounted it is however possible for the medicament to escape out through the conduit and for the plunger to move towards the distal end only by the influence of gravity. In delivery systems such as pump systems, where the conduit is in contact with the fluid medicament for a substantial period of time, it is normal procedure to connect the plunger to the plunger rod in order to provide a controlled forward movement of the plunger thereby preventing the cartridge from emptying itself.

Some infusions pumps available today have a fillable cartridge, which are filled with the medicament prior to use. Such fillable cartridge usually has a plunger having the back wall provided with an interior thread into which a pull rod is screwed. The plunger can then be moved backwards inside the fillable cartridge, and the cartridge can be filled with medicine from a vial. Once the reusable cartridge has been filled, the plunger is disconnected from the pull rod and connected to the pump plunger rod. The assembled cartridge and plunger rod are then inserted into the infusion pump.

It is however desirable to use pre-filled cartridges in pump systems. A major problem in fulfilling this desire is to obtain a suitable connection between the plunger rod of the pump system and the plunger of such pre-filled cartridge.

Different ways of connecting a plunger in a pre-filled cartridge with a plunger rod is disclosed in WO 98/11927.

If the plunger rod is not located at the centre of the plunger, the plunger might tilt once the plunger rod is connected with the plunger rod. When the plunger tilts the sealing between the cartridge wall and the plunger will become leaky.

One way of preventing tilting is, as also disclosed in WO 98/11927, by providing an auxiliary part such as Velcro tape, a magnet or a ball and socket connection at the centre of the plunger. This will ensure that the plunger rod will always connect the plunger at the centre. It is however rather cumbersome from a manufactures point of view and will give rise to a number of failures if the auxiliary part has been disconnected from the plunger prior to use.

When using pre-filled cartridges, it would be preferred if the same pre-filled cartridge can be used both for pump systems and for traditional pen syringes. In pen syringes there are however no connection between the plunger and the plunger rod. Usually the plunger rod has a somewhat flat end, which abuts the plunger. If auxiliary parts are permanently mounted on the plunger, it will be impossible to use the cartridge in an ordinary pen syringe.

An infusion system into which a pre-filled syringe is mounted is disclosed in US 5.928.202. The plunger rod shown in this system is at the distal end provided with a ball-shaped portion and the plunger is provided with a spherical cavity into which the ball-shaped portion can be loaded. The channel of the pre-filled syringe has at the proximal end a funnel-shaped part, which closes the cavity of the plunger around the ball-shaped portion of the plunger rod as the plunger rod is advanced forward. The presence of the funnel-shaped part makes it impracticable to use this solution for a glass cartridge, since these are normally produced with a constant inside diameter, thus it is generally considered an inconvenience to have an uneven outer diameter on a cartridge. The design disclosed in US 5.928.202 does not allow plunger rod disconnection when the plunger is located outside the funnel-shaped zone, and the size of the disconnection force is depended on the exact position of the plunger in the funnel-shaped zone.

In ordinary syringes the plunger is drawn backwards both when the medicament is drawn into the syringe from a vial, and when the injection is being performed to ensure that the needle has not struck a blood vessel. It is therefore a major concern for these types of syringes to ensure that the connection between the plunger and the plunger rod is permanent. Such a connection involving a ball and socket connection which cannot be disconnected is disclosed in EP 395.211, figure 12.

If a ball-shaped portion of a plunger rod is pressed into an interior spherical cavity in a plunger through a circular orifice having a diameter smaller than the diameter of the ball-shaped portion it will be very difficult to disconnect the two parts, and such a connection would henceforth be characterized as permanent.

### Description of the invention:

It is an object of the present invention to provide a disconnectable ball and socket connection which can be used for pre-filled cartridges of the types having a constant inside diameter and which pre-filled cartridges can be used both in pump systems and in ordinary pen type injection systems.

In order to overcome the drawbacks of the prior art it is herein suggested to provide the plunger with an interior cavity engaged by a spherical or semi spherical portion of the plunger rod. According to claim 1 the spherical shaped portion is provided with indentations.

By providing the ball-shaped portion with a number of suitable indentations it has surprisingly been found that the connection can be connected and disconnected using only a limited amount of force, because the indentations allow air in the plunger cavity to escape during plunger rod connection thereby avoiding a counteract pressure build up. The same situation occurs during disconnection as air need to pass through the indentations to avoid counteract under pressure i.e. vacuum in the plunger cavity.

If the diameter of a circular orifice or the minimum cross section of a non-circular orifice has the same diameter as the spherical or semi spherical shaped portion of the plunger rod, a connection, which is very easy to disconnect, is obtained. If however the diameter of the spherical or semi spherical shaped portion of the plunger rod is larger than the minimum cross section or diameter of the open orifice of the plunger as specified in claim 2, the connection will be somewhat tighter.

A suitable disconnectable grip is possible if the outside diameter of the spherical or semi spherical shaped end portion of the plunger rod and the inside diameter of the interior cavity is approximately between 3 to 7 mm and preferably around 5 to 6 mm for a cartridge of 9 to 11 mm, while the diameter of the open orifice should be approximately between 3,5 to 7, mm, preferably around 4 to 5 mm.

The indentations are preferably made such that the spherical or semi spherical shaped portion of the plunger rod has a cross-shaped cross section perpendicular to the longitudinal axis of the plunger rod, as disclosed in claim 3. This provides a suitable connection between the plunger rod and the plunger, where the force needed to disconnect the two parts are determined by the width of the arms of the cross in combination with the diameter of the cross and of the open orifice.

Since bulging of a flexible plunger gives rise to either back suction or after drip when the plunger returns to its initiate form after it has been compressed, bulging should be avoided. In order to minimize bulging of the plunger, which is preferably made from a resilient material such as an elastomeric rubber composition, the point of contact between the plunger rod and the plunger should be located as close to the front wall of the plunger as possible.

It has also been fund that the plunger rod is easier guided into the open orifice if the most distal end of the plunger rod has the shape of a spherical cap such as indicated in claim 4.

Instead of providing the spherical or semi spherical shaped portion of the plunger rod with indentations, the open orifice can be made circular with protrusions, as specified in claim 6. This will also allow air to escape from the cavity during connection, and air to pass into the cavity during disconnection.

When as disclosed in claim 7, the circular cross section of the open orifice has a diameter substantially equal to or larger than the diameter of the spherical or semi spherical shaped portion of the plunger rod and the cross section formed between the protrusions are smaller than the diameter of the spherical or semi spherical shaped portion of the plunger rod it is ensured that the connection is easy disconnectable.

The open orifice could also be provided with a non-circular cross section as specified in claim 9, as this will also allow air to escape from the cavity during connection, and air to pass into the cavity during disconnection.

In order to obtain a good grip, the minimum cross section of the open non-circular orifice should be substantially the same or smaller than the diameter of the spherical or semi spherical portion of the plunger rod, such as cited in claim 10.

The connection between the plunger and the plunger rod as such is claimed in claim 5, 8 and 11.

### Definitions

In the present context, the term "semi spherical" is used to describe an object such as a cavity or an end portion of a plunger having a three-dimensional geometry where at least a part of the surfaces has the shape of a spherical cap.

### Brief Description of the Drawings:

The invention will be explained more fully below in connection with a preferred embodiment and with reference to the drawings in which:
- Figure 1 to 5: Show a sectional view of a plunger according to an embodiment of the invention.
- Figure 6: Show a sectional view of a plunger according to an embodiment of the invention having protrusions.
- Figure 7 to 12: Show a top view of a plunger according to an embodiment of the invention.
- Figure 13: Show a sectional view of a plunger rod according to an embodiment of the invention
- Figure 14: Show a sectional view of a plunger rod according to an embodiment of the invention having a cross-shaped end portion.
- Figure 15 to 18: Show a sectional view of a plunger rod according to an embodiment of the invention having different forms of apertures.
- Figure 19-20: Show a sectional view of a plunger rod according to an embodiment of the invention having outwardly pointing protrusions.
- Figure 21: Show a sectional view of a plunger rod according to an embodiment of the invention having slots.

The figures are schematic and simplified for clarity, and they just show details, which are essential to the understanding of the invention, while other details are left out. Throughout, the same reference numerals are used for identical or corresponding parts.

### Detailed Description of Embodiment:

Some preferred embodiments have been shown in the foregoing, but it should be stressed that the invention is not limited to these, but may be embodied in other ways within the subject matter defined in the following claims.

Initially it may be convenient to define that the term "distal end" is meant to refer to the end of cartridge where the fluid medicament is contained and where the conduit is inserted, whereas the term "proximal end" is meant to refer to the opposite end where the plunger is located in its initial position.

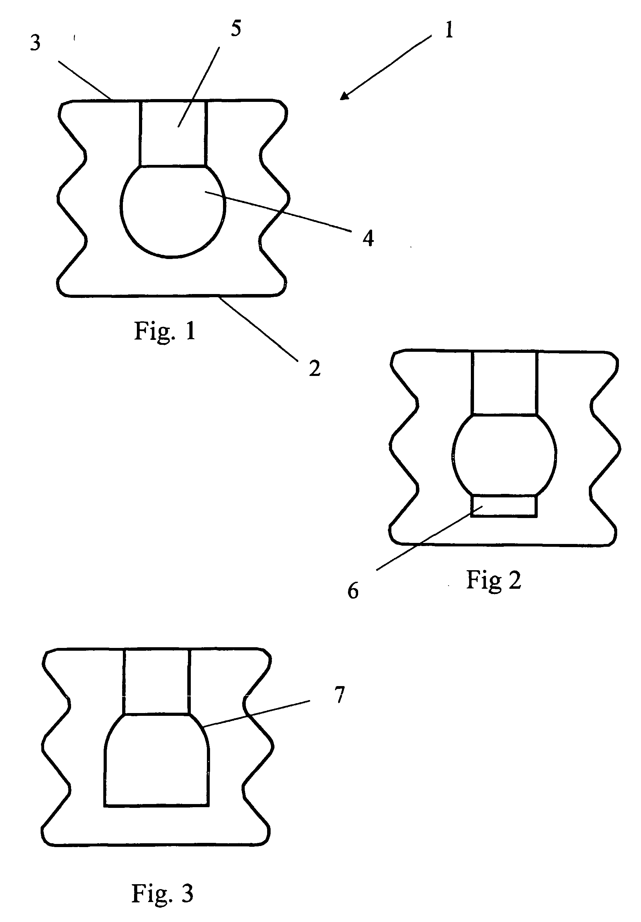

Figure 1-6 shows different embodiments of a plunger 1 as seen from the side.

The plunger 1 illustrated in figure 1 has a front wall 2, which is in contact with the fluid medicament inside the cartridge and an opposite located back wall 3. Encapsulated in the interior of the plunger 1 is a spherical cavity 4 provided. This cavity 4 is connected to the back wall 3 through an open orifice 5.

In the embodiment disclosed in figure 2, the cavity 4 is prolonged in the distal direction with an additional cylindrical appendix 6.

The cavity 4 shown in figure 3 has only a semi spherical shape, as the proximal end 7 of the cavity 4 has the shape of a spherical cap, while the distal end of the cavity has a cylindrical form.

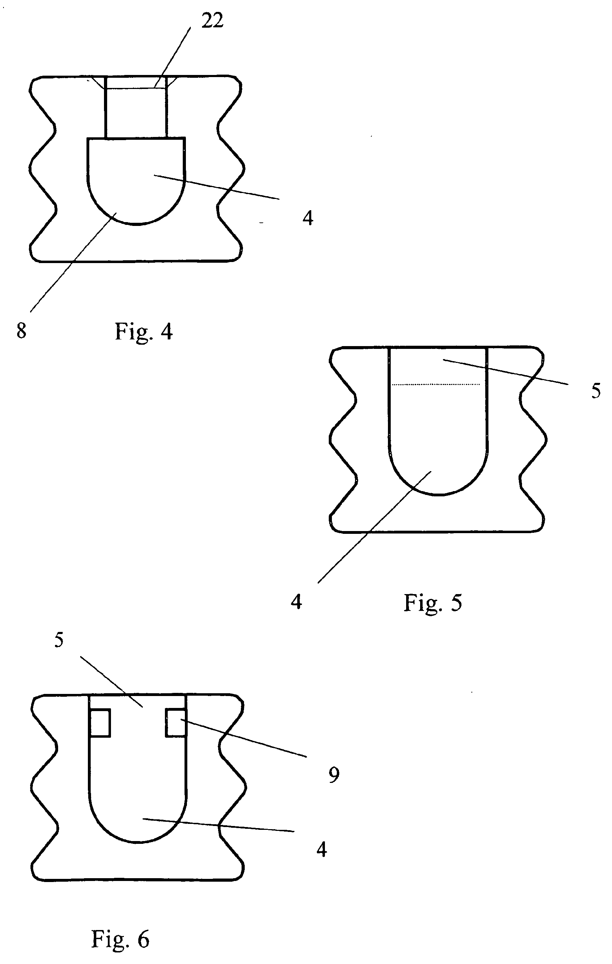

Figure 4 also shows a semi spherical cavity 4 having the shape of a spherical cap at the distal end 8, while the proximal end has a cylindrical shape. The proximal end of the open orifice 5 could be provided with an inlet 22, which makes it easier to load the plunger rod 6 into the open orifice 5.

The plunger illustrated in figure 5 has a cavity 4 similar to the cavity 4 shown in figure 4, however the open orifice has a diameter equal to the diameter of both the cylindrical part and the spherical cap part.

In order to enhance the grip between the plunger 1 and the plunger rod 6 in the embodiment shown in figure 5, the open orifice 5 could be provided with protrusions 9 as illustrated in figure 6. The number and size of the protrusions 9 can be chosen randomly in order to provide the grip needed for a particular injection or infusion system.

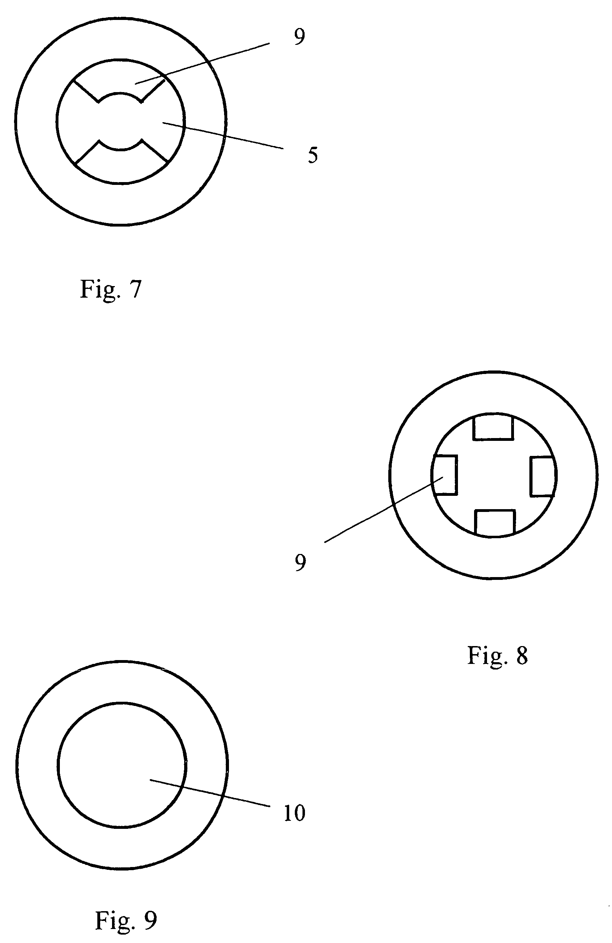

Figure 7 shows a top view of a plunger with two such protrusions 9, and figure 8 shows a plunger with four protrusions 9.

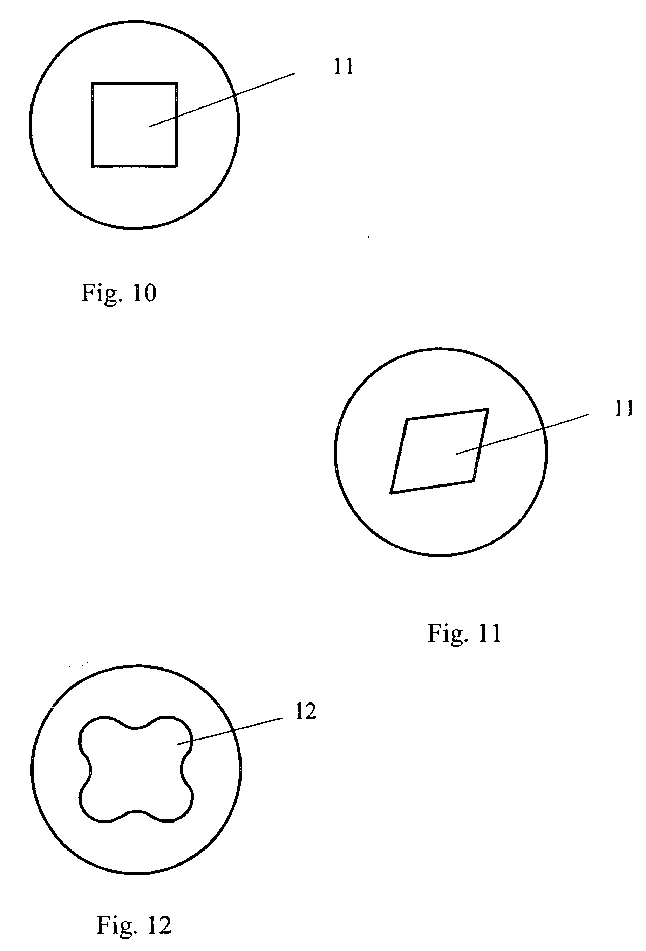

The open orifice 5 could have a substantially circular cross section 10 as shown in figure 9, or the cross section could be four-sided 11 as shown in figure 10 and figure 11. The non-circular cross section of the open orifice 5 could in fact be any kind of polygon 12 as indicated in figure 12, or even any other shape e.g. shaped like a four-leaf.

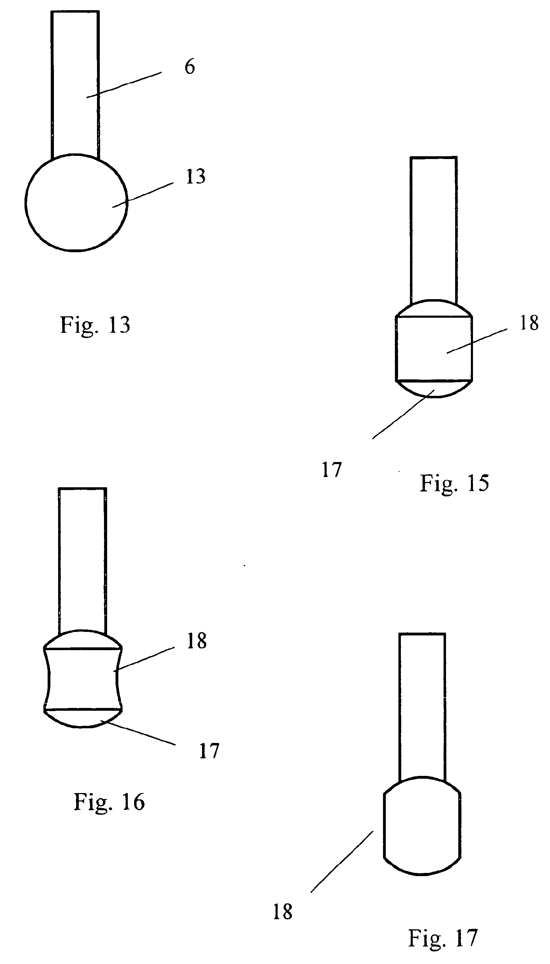

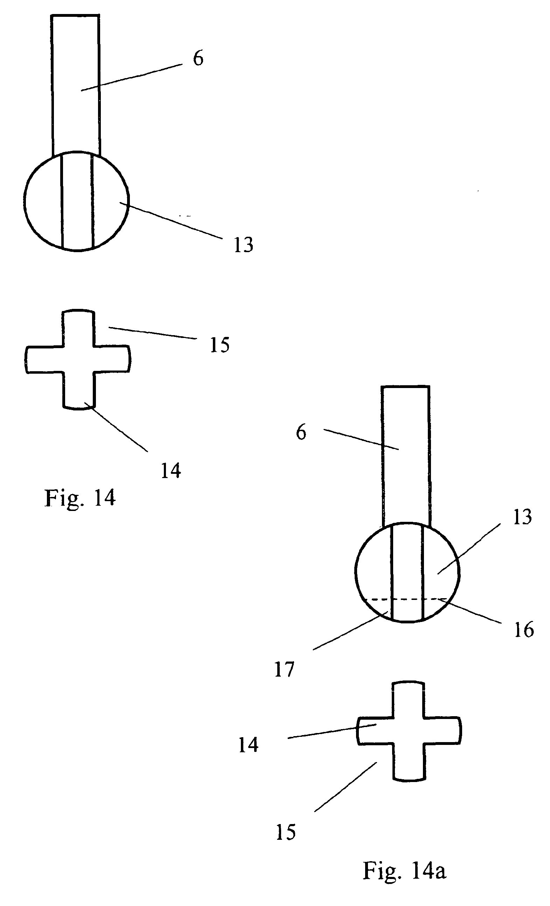

The plunger rod 6 of the connection is shown in figure 13-20. When the spherical or semi spherical end portion 13 of the plunger rod 6 is inserted into the cavity 4 of the plunger 1 a hipbone connection is established. Due to this hipbone connection a plunger rod 6 with a diameter substantially smaller than the diameter of the cartridge can be used without tilting the plunger 1 if the plunger rod 6 is pressed towards the side of the cartridge. When the plunger 1 and the plunger rod 13 are connected in a stiff or permanent connection, the plunger might tilt and cause leakage if the plunger rod 13 is not kept in the centre of the cartridge, a hipbone connection however solves this problem.

Figure 13 illustrates a plunger rod 6 with a spherical shaped end portion 13 located at the distal end of the plunger rod 6. In order to obtain a disconnectable connection this spherical shaped portion 13 is preferably provided with tracks, slots, apertures or similar indentations as illustrated in figure 14-18.

In the embodiment disclosed in figure 14, the spherical shaped end portion 13 of the plunger rod 6 has a cross-shaped cross section when viewed perpendicular to the longitudinal axis of the plunger rod 6. The cross 14 has four arms, which arms each abuts the imaginary diameter of the spherical end portion 13. The space 15 between the arms can off cause vary in size, which is also the case for the number of arms.

Both figure 14 and figure 14a shows a schematic view of the cross section of the spherical part 13.

The space 15 needs not extend through the full longitudinal diameter of the spherical shaped portion 13, but could end at a line 16 leaving a spherical cap 17 at the most distal end of the spherical end portion 13, as shown in figure 14a.

A number of different plunger rods 6 are disclosed in figure 15-17. These plunger rods 6 has a spherical portion 13 at the distal end with indentations 18. These indentations all leave a spherical cap 17 at the most distal end of the plunger rod 6.

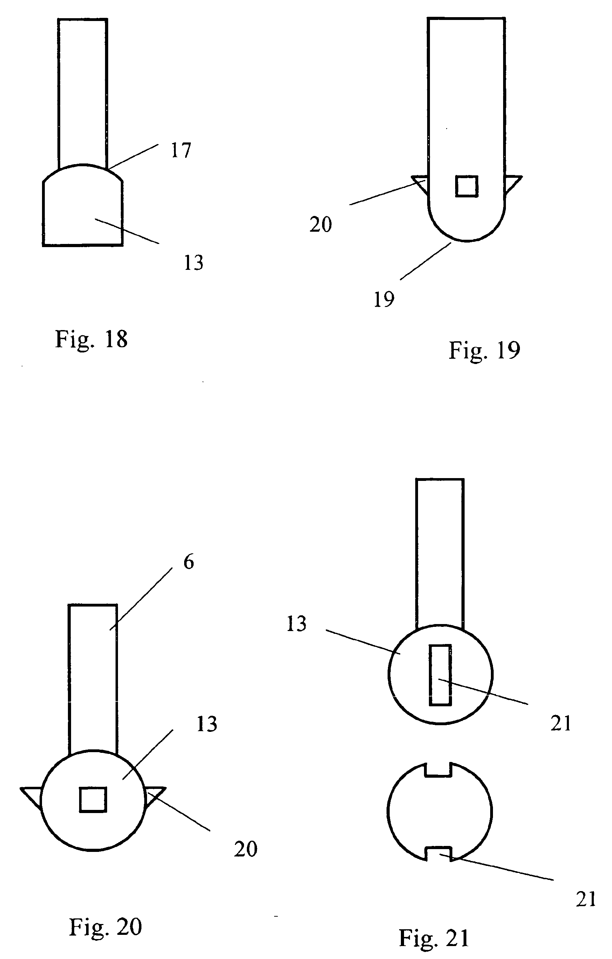

Figure 18 shows a plunger rod 6 where the spherical portion 13 is provided with indentations leaving a spherical cap 17 at the proximal end of the spherical end portion 13

The distal end of the plunger rod 6 could also, as shown in figure 19, be provided with a semi spherical end portion 19 which could be provided with outwardly pointing protrusions 20 in order to enhance the grip. Such protrusions 20 could also be provided at the spherical end portion 13 of the plunger rod 6 as shown in figure 20.

Figure 21 shows a plunger rod 6 with a spherical end portion 13 having two slots 21. A schematic view of the cross section of the spherical part 13 is also included.

Some preferred embodiments have been shown in the foregoing, but it should be stressed that the invention is not limited to these, but may be embodied in other ways within the subject matter defined in the following claims.

## Claims

1. A medical delivery system comprising:
a cartridge with a distal end closed by a flexible membrane and a proximal end connected by a wall forming a vessel containing a liquid medicament,
a flexible plunger (1) received in the proximal end of the vessel and moved along the vessel in order to expel the fluid medicament, the flexible plunger (1) having a front wall (2) in contact with the fluid medicament and an opposite back wall (3), which back wall (3) is provided with a spherical or semi spherical shaped cavity (4) fully encapsulated in the interior of the flexible plunger (1) and connected to the back wall (3) of the flexible plunger (1) through an open orifice (5),
a plunger rod (6) which can be moved forward inside the vessel in order to move the flexible plunger (1) forward and which plunger rod (6) is disconnectable connected to the flexible plunger (1),
the disconnectable connection between the flexible plunger (1) and the plunger rod (6) being obtained by a spherical or semi spherical portion (13, 19) provided at a distal end of the plunger rod (6) engaging the spherical or semi spherical shaped cavity (4) of the flexible plunger (1),
**characterized in that**,
the spherical or semi spherical shaped portion (13, 19) is provided with tracks, slots or apertures (15, 18, 21) allowing air to escape during connection.

2. A medical delivery system according to claim 1, **characterized in that**, the diameter of the spherical or semi spherical shaped portion (13.19) of the plunger rod (6) is larger than the minimum cross section or diameter of the open orifice (5) of the flexible plunger (1).

3. A medical delivery system according to claim 1 or 2, **characterized in that**, the spherical or semi spherical shaped portion (13, 19) has a cross-shaped cross section perpendicular to the longitudinal axis of the plunger rod (6).

4. A medical delivery system according to anyone of the claims 1 to 3, **characterized in that**, the most distal end of the spherical or semi spherical shaped portion (13, 19) has the shape of a spherical cap (17).

5. A connection between a flexible plunger (1) and a plunger rod (6), which comprises in combination;
a flexible plunger (1) with a front wall (2) and a back wall (3), the flexible plunger (1) having an interior spherical or semi spherical shaped cavity (4) connected to the back wall (3) through an open orifice (5),
a plunger rod (6) with a spherical or semi spherical shaped end portion (13, 19), which end portion (13, 19) disconnectable engages the cavity (4) of the flexible plunger (1), such that the flexible plunger (1) and the plunger rod (6) moves together,
**characterized in that**, the spherical or semi spherical shaped end portion (13, 19) of the plunger rod (6) is provided with tracks, slots or apertures (15, 18, 21) allowing air to escape during connection.

6. A medical delivery system comprising:
a cartridge with a distal end closed by a flexible membrane and a proximal end connected by a wall forming a vessel containing a liquid medicament,
a flexible plunger (1) received in the proximal end of the vessel and moved along the vessel in order to expel the fluid medicament, the flexible plunger (1) having a front wall (2) in contact with the fluid medicament and an opposite back wall (3), which back wall (3) is provided with a spherical or semi spherical shaped cavity (4) fully encapsulated in the interior of the flexible plunger (1) and connected to the back wall (3) of the flexible plunger (1) through an open orifice (5),
a plunger rod (6) which can be moved forward inside the vessel in order to move the flexible plunger (1) forward and which plunger rod (6) is disconnectable connected to the flexible plunger (1),
the disconnectable connection between the flexible plunger (1) and the plunger rod (6) being obtained by a spherical or semi spherical portion (13, 19) provided at a distal end of the plunger rod (6) engaging the spherical or semi spherical shaped cavity (4) of the flexible plunger (1),
**characterized in that**, the open orifice (5) has a substantially circular cross section (10) provided with protrusions (9) allowing air to escape during connection.

7. A medical delivery system according to claim 6, **characterized in that**, the circular cross section of the open orifice (5) has a diameter substantially equal to or larger than the diameter of the spherical or semi spherical shaped portion (13) of the plunger rod (6) and that the cross section formed between the protrusions (9) are smaller than the diameter of the spherical or semi spherical shaped portion (13) of the plunger rod (6).

8. A connection between a flexible plunger (1) and a plunger rod (6), which comprises in combination
a flexible plunger (1) with a front wall (2) and a back wall (3), the flexible plunger (1) having an interior spherical or semi spherical shaped cavity (4) connected to the back wall (3) through an open orifice (5),
a plunger rod (6) with a spherical or semi spherical shaped end portion (13, 19), which end portion (13. 19) disconnectable engages the cavity (4) of the flexible plunger (1), such that the flexible plunger (1) and the plunger rod (6) moves together,
**characterized in that** the open orifice (5) has a substantially circular cross section (10) provided with protrusions (9) allowing air to escape during connection.

9. A medical delivery system comprising:
a cartridge with a distal end closed by a flexible membrane and a proximal end connected by a wall forming a vessel containing a liquid medicament,
a flexible plunger (1) received in the proximal end of the vessel and moved along the vessel in order to expel the fluid medicament, the flexible plunger (1) having a front wall (2) in contact with the fluid medicament and an opposite back wall (3), which back wall (3) is provided with a spherical or semi spherical shaped cavity (4) fully encapsulated in the interior of the flexible plunger (1) and connected to the back wall (3) of the flexible plunger (1) through an open orifice (5),
a plunger rod (6) which can be moved forward inside the vessel in order to move the flexible plunger forward and which plunger rod (6) is disconnectable connected to the flexible plunger (1),
the disconnectable connection between the flexible plunger (1) and the plunger rod (6) being obtained by a spherical or semi spherical portion (13, 19) provided at a distal end of the plunger rod (6) engaging the spherical shaped cavity (4) of the flexible plunger (1),
**characterized in that**, the open orifice (5) has a non-circular cross section (11, 12) allowing air to escape during connection.

10. A medical delivery system according to claim 9, **characterized in that**, the minimum cross section of the orifice (5) is substantially the same or smaller than the diameter of the spherical shaped or semi spherical portion (13, 19) of the plunger rod (6).

11. A connection between a flexible plunger (1) and a plunger rod (6), which comprises in combination
a flexible plunger (1) with a front wall (2) and a back wall (3), the flexible plunger (1) having an interior spherical shaped cavity (4) connected to the back wall (3) through an open orifice (5),
a plunger rod (6) with a spherical or semi spherical shaped end portion (13, 19), which end portion (13) disconnectable engages the cavity (4) of the flexible plunger (1), such that the flexible plunger (1) and the plunger rod (6) moves together,
**characterized in that** the open orifice (5) has a non-circular cross section (11, 12) allowing air to escape during connection.

## Patentansprüche

1. Medikamentenabgabesystem, umfassend:
eine Patrone mit einem durch eine flexible Membran verschlossenen entfernten Ende und einem nahen Ende, das durch eine Wand verbunden ist, die ein flüssiges Arzneimittel enthaltendes Gefäß bildet,
einen flexiblen Kolben (1), der im nahen Ende des Gefäßes aufgenommen ist und entlang des Gefäßes bewegt wird, um das flüssige Medikament auszustoßen, wobei der flexible Kolben (1) eine Vorderwand (2) in Kontakt mit dem flüssigen Medikament und eine gegenüber liegende Rückwand (3) aufweist, wobei die Rückwand (3) mit einem kugelförmig oder halbkugelförmig gestalteten Hohlraum (4) vollständig im Inneren des flexiblen Kolbens (1) eingekapselt bereitgestellt und mit der Rückwand (3) des flexiblen Kolbens (1) durch eine offene Öffnung (5) verbunden ist,
eine Kolbenstange (6) die vorwärts in das Gefäß bewegt werden kann, um den flexiblen Kolben (1) vorwärts zu bewegen, und wobei die Kolbenstange (6) mit dem flexiblen Kolben (1) trennbar verbunden ist,
wobei die trennbare Verbindung zwischen dem flexiblen Kolben (1) und der Kolbenstange (6) durch einen am entfernten Ende der Kolbenstange (6) bereitgestellten kugelförmigen oder halbkugelförmigen Teil (13, 19) erhalten wird, der mit dem kugelförmig oder halbkugelförmig gestalteten Hohlraum (4) des flexiblen Kolbens (1) in Eingriff steht,
**dadurch gekennzeichnet, dass**
der kugelförmig oder halbkugelförmig gestaltete Teil (13, 19) mit Bahnen, Schlitzen oder Ausschnitten (15, 18, 21) bereitgestellt ist, die es ermöglichen, dass Luft während des Verbindens entweicht.

2. Medikamentenabgabesystem nach Anspruch 1, **dadurch gekennzeichnet, dass** der Durchmesser des kugelförmig oder halbkugelförmig gestalteten Teils (13, 19) der Kolbenstange (6) größer ist als der Mindestquerschnitt oder -durchmesser der offenen Öffnung (5) des flexiblen Kolbens (1).

3. Medikamentenabgabesystem nach Anspruch 1 oder 2, **dadurch gekennzeichnet, dass** der kugelförmig oder halbkugelförmig gestaltete Teil (13, 19) einen zur Längsachse der Kolbenstange (6) senkrechten kreuzförmigen Querschnitt aufweist.

4. Medikamentenabgabesystem nach einem der Ansprüche 1 bis 3, **dadurch gekennzeichnet, dass** das am weitesten entfernte Ende des kugelförmig oder halbkugelförmig gestalteten Teils (13, 19) die Gestalt einer kugelförmigen Kappe (17) aufweist.

5. Verbindung zwischen einem flexiblen Kolben (1) und einer Kolbenstange (6), die in Kombination umfassen:
einen flexiblen Kolben (1) mit einer Vorderwand (2) und einer Rückwand (3), wobei der flexible Kolben (1) einen mit der Rückwand (3) durch eine offene Öffnung (5) verbundenen inneren kugelförmig oder halbkugelförmig gestalteten Hohlraum (4) aufweist,
eine Kolbenstange (6) mit einem kugelförmig oder halbkugelförmig gestalteten Endteil (13, 19), wobei der Endteil (13, 19) trennbar mit dem Hohlraum (4) des flexiblen Kolbens (1) in Eingriff steht, so dass sich der flexible Kolben (1) und die Kolbenstange (6) gemeinsam bewegen,
**dadurch gekennzeichnet, dass** der kugelförmig oder halbkugelförmig gestaltete Endteil (13, 19) der Kolbenstange (6) mit Bahnen, Schlitzen oder Ausschnitten (15, 18, 21) bereitgestellt ist, die es ermöglichen, dass Luft während des Verbindens entweicht.

6. Medikamentenabgabesystem, umfassend:
eine Patrone mit einem durch eine flexible Membran verschlossenen entfernten Ende und einem nahen Ende, das durch eine Wand verbunden ist, die ein ein flüssiges Arzneimittel enthaltendes Gefäß bildet,
einen flexiblen Kolben (1), der im nahen Ende des Gefäßes aufgenommen ist und entlang des Gefäßes bewegt wird, um das flüssige Medikament auszustoßen, wobei der flexible Kolben (1) eine Vorderwand (2) in Kontakt mit dem flüssigen Medikament und eine gegenüber liegende Rückwand (3) aufweist, wobei die Rückwand (3) mit einem kugelförmig oder halbkugelförmig gestalteten Hohlraum (4) vollständig im Inneren des flexiblen Kolbens (1) eingekapselt bereitgestellt und mit der Rückwand (3) des flexiblen Kolbens (1) durch eine offene Öffnung (5) verbunden ist,
eine Kolbenstange (6) die vorwärts in das Gefäß bewegt werden kann, um den flexiblen Kolben (1) vorwärts zu bewegen, und wobei Kolbenstange (6) mit dem flexiblen Kolben (1) trennbar verbunden ist,
wobei die trennbare Verbindung zwischen dem flexiblen Kolben (1) und der Kolbenstange (6) durch einen am entfernten Ende der Kolbenstange (6) bereitgestellten kugelförmigen oder halbkugelförmigen Teil (13, 19) erhalten wird, der mit dem kugelförmig oder halbkugelförmig gestalteten Hohlraum (4) des flexiblen Kolbens (1) in Eingriff steht,
**dadurch gekennzeichnet, dass** die offene Öffnung (5) einen mit Vorsprüngen bereitgestellten im Wesentlichen kreisförmigen Querschnitt (10) aufweist, der es ermöglicht, dass Luft während des Verbindens entweicht.

7. Medikamentenabgabesystem nach Anspruch 6, **dadurch gekennzeichnet, dass** der kreisförmige Querschnitt der offenen Öffnung (5) einen Durchmesser aufweist, der gleich oder größer als der Durchmesser des kugelförmig oder halbkugelförmig gestalteten Teils (13) der Kolbenstange (6) ist, und dass der zwischen den Vorsprüngen (9) gestaltete Querschnitt kleiner als der Durchmesser des kugelförmig oder halbkugelförmig gestalteten Teils (13) der Kolbenstange (6) ist.

8. Verbindung zwischen einem flexiblen Kolben (1) und einer Kolbenstange (6), die in Kombination umfassen:
einen flexiblen Kolben (1) mit einer Vorderwand (2) und einer Rückwand (3), wobei der flexible Kolben (1) einen mit der Rückwand (3) durch eine offene Öffnung (5) verbundenen inneren kugelförmig oder halbkugelförmig gestalteten Hohlraum (4) aufweist,
eine Kolbenstange (6) mit einem kugelförmig oder halbkugelförmig gestalteten Endteil (13, 19), wobei Endteil (13, 19) trennbar mit dem Hohlraum (4) des flexiblen Kolbens (1) in Eingriff steht, so dass sich der flexible Kolben (1) und die Kolbenstange (6) gemeinsam bewegen,
**dadurch gekennzeichnet, dass** die offene Öffnung (5) einen mit Vorsprüngen bereitgestellten im Wesentlichen kreisförmigen Querschnitt (10) aufweist, der es ermöglicht, dass Luft während des Verbindens entweicht.

9. Medikamentenabgabesystem, umfassend:
eine Patrone mit einem durch eine flexible Membran geschlossenen entfernten Ende und einem nahen Ende, das mit einer Wand verbunden ist, die ein ein flüssiges Arzneimittel enthaltendes Gefäß bildet,
einen flexiblen Kolben (1), der im nahen Ende des Gefäßes aufgenommen ist und entlang des Gefäßes bewegt wird, um das flüssige Medikament auszustoßen, wobei der flexible Kolben (1) eine Vorderwand (2) in Kontakt mit dem flüssigen Medikament und eine gegenüber liegende Rückwand (3) aufweist, wobei die Rückwand (3) mit einem kugelförmig oder halbkugelförmig gestalteten Hohlraum (4) vollständig im Inneren des flexiblen Kolbens (1) eingekapselt bereitgestellt und mit der Rückwand (3) des flexiblen Kolbens (1) durch eine offene Öffnung (5) verbunden ist,
eine Kolbenstange (6) die vorwärts in das Gefäß bewegt werden kann, um den flexiblen Kolben vorwärts zu bewegen und welche Kolbenstange (6) mit dem flexiblen Kolben (1) trennbar verbunden ist,
wobei die trennbare Verbindung zwischen dem flexiblen Kolben (1) und der Kolbenstange (6) durch einen am entfernten Ende der Kolbenstange (6) bereitgestellten kugelförmigen oder halbkugelförmigen Teil (13, 19) erhalten wird, der mit dem kugelförmig gestalteten Hohlraum (4) des flexiblen Kolbens (1) in Eingriff steht,
**dadurch gekennzeichnet, dass** die offene Öffnung (5) einen nichtkreisförmigen Querschnitt (11, 12) aufweist, der es ermöglicht, dass Luft während des Verbindens entweicht.

10. Medikamentenabgabesystem nach Anspruch 9, **dadurch gekennzeichnet, dass** der Mindestquerschnitt der Öffnung (5) im Wesentlichen der gleiche oder kleiner als der Durchmesser des kugelförmig oder halbkugelförmig gestalteten Teils (13, 19) der Kolbenstange (6) ist.

11. Verbindung zwischen einem flexiblen Kolben (1) und einer Kolbenstange (6), die in Kombination umfassen:
einen flexiblen Kolben (1) mit einer Vorderwand (2) und einer Rückwand (3), wobei der flexible Kolben (1) einen mit der Rückwand (3) durch eine offene Öffnung (5) verbundenen inneren kugelförmigen Hohlraum (4) aufweist,
eine Kolbenstange (6) mit einem kugelförmig oder halbkugelförmig gestalteten Endteil (13, 19), wobei Endteil (13) trennbar mit dem Hohlraum (4) des flexiblen Kolbens (1) in Eingriff steht, so dass sich der flexible Kolben (1) und die Kolbenstange (6) gemeinsam bewegen,
**dadurch gekennzeichnet, dass** die offene Öffnung (5) einen nichtkreisförmigen Querschnitt (11, 12) aufweist, der es ermöglicht, dass Luft während des Verbindens entweicht.

## Revendications

1. Système d'administration de médicament, comprenant :
une cartouche présentant une extrémité distale fermée par une membrane souple, et une extrémité proximale reliée par une paroi, définissant un récipient contenant un médicament liquide,
un piston souple (1) logé dans l'extrémité proximale du récipient et déplacé le long du récipient afin d'expulser le médicament fluide, le piston souple (1) présentant une paroi frontale (2) en contact avec le médicament fluide et une paroi arrière (3) opposée, laquelle paroi arrière (3) est pourvue d'une cavité de forme sphérique ou hémisphérique (4) complètement enfermée à l'intérieur du piston souple (1) et reliée à la paroi arrière (3) du piston souple (1) par l'intermédiaire d'un orifice ouvert (5),
une tige de piston (6) qui peut être déplacée vers l'avant à l'intérieur du récipient afin de déplacer le piston souple (1) vers l'avant, laquelle tige de piston (6) est reliée de manière libérable au piston souple (1),
la liaison libérable entre le piston souple (1) et la tige de piston (6) étant obtenue par une partie sphérique ou hémisphérique (13, 19) prévue au niveau d'une extrémité distale de la tige de piston (6) et pénétrant dans la cavité de forme sphérique ou hémisphérique (4) du piston souple (1),
**caractérisé en ce que**
la partie de forme sphérique ou hémisphérique (13, 19) est pourvue de voies, de fentes ou d'orifices (15, 18, 21) permettant un échappement d'air pendant la liaison.

2. Système d'administration de médicament selon la revendication 1, **caractérisé en ce que** le diamètre de la partie de forme sphérique ou hémisphérique (13, 19) de la tige de piston (6) est plus grand que la section transversale minimale ou diamètre minimal de l'orifice ouvert (5) du piston souple (1).

3. Système d'administration de médicament selon la revendication 1 ou 2, **caractérisé en ce que** la partie de forme sphérique ou hémisphérique (13, 19) a une section transversale en croix, perpendiculairement à l'axe longitudinal de la tige de piston (6).

4. Système d'administration de médicament selon l'une quelconque des revendications 1 à 3, **caractérisé en ce que** l'extrémité distale extrême de la partie de forme sphérique ou hémisphérique (13, 19) a la forme d'une calotte sphérique (17).

5. Liaison entre un piston souple (1) et une tige de piston (6), comprenant en combinaison :
un piston souple (1) présentant une paroi frontale (2) et une paroi arrière (3), le piston souple (1) comportant une cavité intérieure de forme sphérique ou hémisphérique (4), reliée à la paroi arrière (3) par l'intermédiaire d'un orifice ouvert (5),
une tige de piston (6) présentant une partie d'extrémité de forme sphérique ou hémisphérique (13, 19), laquelle partie d'extrémité (13, 19) pénètre de manière libérable dans la cavité (4) du piston souple (1), si bien que le piston souple (1) et la tige de piston (6) se déplacent ensemble,
**caractérisé en ce que** la partie d'extrémité de forme sphérique ou hémisphérique (13, 19) de la tige de piston (6) est pourvue de voies, de fentes ou d'orifices (15, 18, 21) permettant un échappement d'air pendant la liaison.

6. Système d'administration de médicament, comprenant :
une cartouche présentant une extrémité distale fermée par une membrane souple, et une extrémité proximale reliée par une paroi, définissant un récipient contenant un médicament liquide,
un piston souple (1) logé dans l'extrémité proximale du récipient et déplacé le long du récipient afin d'expulser le médicament fluide, le piston souple (1) présentant une paroi frontale (2) en contact avec le médicament fluide et une paroi arrière (3) opposée, laquelle paroi arrière (3) est pourvue d'une cavité de forme sphérique ou hémisphérique (4) complètement enfermée à l'intérieur du piston souple (1) et reliée à la paroi arrière (3) du piston souple (1) par l'intermédiaire d'un orifice ouvert (5),
une tige de piston (6) qui peut être déplacée vers l'avant à l'intérieur du récipient afin de déplacer le piston souple (1) vers l'avant, laquelle tige de piston (6) est reliée de manière libérable au piston souple (1),
la liaison libérable entre le piston souple (1) et la tige de piston (6) étant obtenue par une partie sphérique ou hémisphérique (13, 19) prévue au niveau d'une extrémité distale de la tige de piston (6) et pénétrant dans la cavité de forme sphérique ou hémisphérique (4) du piston souple (1),
**caractérisé en ce que** l'orifice ouvert (5) présente une section transversale sensiblement circulaire (10) pourvue de protubérances (9) permettant un échappement d'air pendant la liaison.

7. Système d'administration de médicament selon la revendication 6, **caractérisé en ce que** la section transversale circulaire de l'orifice ouvert (5) a un diamètre sensiblement égal ou supérieur au diamètre de la partie de forme sphérique ou hémisphérique (13) de la tige de piston (6), et **en ce que** la section transversale formée entre les protubérances (9) est plus petite que le diamètre de la partie de forme sphérique ou hémisphérique (13) de la tige de piston (6).

8. Liaison entre un piston souple (1) et une tige de piston (6), comprenant en combinaison :
un piston souple (1) présentant une paroi frontale (2) et une paroi arrière (3), le piston souple (1) comportant une cavité intérieure de forme sphérique ou hémisphérique (4), reliée à la paroi arrière (3) par l'intermédiaire d'un orifice ouvert (5),
une tige de piston (6) présentant une partie d'extrémité de forme sphérique ou hémisphérique (13, 19), laquelle partie d'extrémité (13, 19) pénètre de manière libérable dans la cavité (4) du piston souple (1), si bien que le piston souple (1) et la tige de piston (6) se déplacent ensemble,
**caractérisé en ce que** l'orifice ouvert (5) a une section transversale sensiblement circulaire (10) pourvue de protubérances (9) permettant un échappement d'air pendant la liaison.

9. Système d'administration de médicament, comprenant :
une cartouche présentant une extrémité distale fermée par une membrane souple, et une extrémité proximale reliée par une paroi, définissant un récipient contenant un médicament liquide,
un piston souple (1) logé dans l'extrémité proximale du récipient et déplacé le long du récipient afin d'expulser le médicament fluide, le piston souple (1) présentant une paroi frontale (2) en contact avec le médicament fluide et une paroi arrière (3) opposée, laquelle paroi arrière (3) est pourvue d'une cavité de forme sphérique ou hémisphérique (4) complètement enfermée à l'intérieur du piston souple (1) et reliée à la paroi arrière (3) du piston souple (1) par l'intermédiaire d'un orifice ouvert (5),
une tige de piston (6) qui peut être déplacée vers l'avant à l'intérieur du récipient afin de déplacer le piston souple vers l'avant, laquelle tige de piston (6) est reliée de manière libérable au piston souple (1),
la liaison libérable entre le piston souple (1) et la tige de piston (6) étant obtenue par une partie sphérique ou hémisphérique (13, 19) prévue au niveau d'une extrémité distale de la tige de piston (6) et pénétrant dans la cavité de forme sphérique (4) du piston souple (1),
**caractérisé en ce que** l'orifice ouvert (5) présente une section transversale non circulaire (11, 12) permettant un échappement d'air pendant la liaison.

10. Système d'administration de médicament selon la revendication 9, **caractérisé en ce que** la section transversale minimale de l'orifice (5) est sensiblement égale ou inférieure au diamètre de la partie de forme sphérique ou hémisphérique (13, 19) de la tige de piston (6).

11. Liaison entre un piston souple (1) et une tige de piston (6), comprenant en combinaison :
un piston souple (1) présentant une paroi frontale (2) et une paroi arrière (3), le piston souple (1) comportant une cavité intérieure de forme sphérique (4), reliée à la paroi arrière (3) par l'intermédiaire d'un orifice ouvert (5),
une tige de piston (6) présentant une partie d'extrémité de forme sphérique ou hémisphérique (13, 19), laquelle partie d'extrémité (13) pénètre de manière libérable dans la cavité (4) du piston souple (1), si bien que le piston souple (1) et la tige de piston (6) se déplacent ensemble,
**caractérisé en ce que** l'orifice ouvert (5) a une section transversale non circulaire (11, 12) permettant un échappement d'air pendant la liaison.
